# EUROPEAN PATENT APPLICATION

(11) **EP 4 339 959 A1**
(43) Date of publication of application: **20.03.2024**
(21) Application number: 23197093.0
(22) Date of filing: 13.09.2023
(51) Int. Cl.: G16C 20/30

(54) **SOLUBILITY EVALUATION DEVICE, SOLUBILITY EVALUATION METHOD, AND SOLUBILITY EVALUATION PROGRAM**

(30) Priority: 16.09.2022 JP 2022148363
(71) Applicant: Yokogawa Electric Corporation, Tokyo 180-8750 (JP)
(72) Inventor: IMAMURA, Yusuke, Musashino-shi, Tokyo, 180-8750 (JP); MATUBAYASI, Nobuyuki, Osaka, 560-8531 (JP)
(74) Representative: Winter, Brandl - Partnerschaft mbB

(57) **Abstract**

An initial conformation setting unit 11 sets multiple different initial conformations. a solubility feature calculation unit 13 calculates a solubility feature for each molecular simulation for each initial conformation. A partial three-dimensional structural information acquisition unit 14 calculates partial three-dimensional structural information of a molecule in each molecular simulation. Based on a solubility feature and partial three-dimensional structural information for each molecular simulation, a partial structure specification unit 15 specifies a partial structure corresponding to variation in the solubility features. A variation evaluation unit 17 selects a group based on the thermodynamic stability of each group in which the solubility feature is classified based on the partial structure, and calculates variation in the solubility features. When the variation is equal to or greater than a target variation value, a variation determination unit 18 causes multiple new initial conformations to be set. An output unit 19 outputs the evaluation results when the variation is less than the target variation value.

## Description

### FIELD

The present invention relates to a solubility evaluation device, a solubility evaluation method, and a solubility evaluation program.

### BACKGROUND

In general, molecular simulations used in molecular dynamics depend on the initial structure and initial conformation of the molecule. Hence, different simulation results may be obtained due to differences in the initial structure and initial conformation. One of the reasons may be due to the difference in the local stability of a system balanced by initial conformation. Hence, it may be that the tree-dimensional structure and energy state sampled during simulation vary due to the initial structure. Therefore, to present the calculation results of molecular simulation, it is preferable to present the simulation results while taking into account the variation in the results caused by the initial conformation.

Moreover, in the molecular dynamics method, it is possible to reduce initial conformational dependence of the calculation, using a method of shifting to a locally stable state that is more stable, including a heat annealing method, and a method for improving efficiency of sampling such as a replica exchange method. On the other hand, even if these methods are implemented, it is still important to evaluate variations in the results of different initial conformations in the final calculation results, to demonstrate the reliability of the calculation results, and the robustness of the calculation model and the procedure.

For example, as a simulation technique in computational chemistry, a method for simulating the breakage of molecular chains of a polymer material has been developed (for example, Japanese Patent Application Laid-open No. 2018-101354). Moreover, a physical property value calculation device having a function of calculating stress data, relaxation modulus, and viscoelasticity from the time series data of the molecular dynamics calculation using a polymer model has been developed (Japanese Laid-open Patent Publication No. 2015-203682). These techniques evaluate physical properties using the results of molecular simulation. Moreover, a technique for improving the accuracy of thermal conductivity calculation using the time average value and temporal fluctuation of the molecular dynamics simulation results has been developed (Japanese Laid-open Patent Publication No. 2005-233752). Furthermore, a technique for evaluating and classifying the calculation results of molecular dynamics for medicine at high speed and high accuracy has been developed (U.S. Patent No. 2021/0375399). In this technology, analysis on the results of the molecular dynamics is made efficient by causing the Support Vector Machine (SVM) to study the Root Mean Square Deviation (RMSD) of the three-dimensional structure of the molecule and labeled calculation results.

However, with the conventional molecular simulation techniques, it is difficult to appropriately evaluate the initial conformation, and suppress the variation in the calculation results of solubility features due to differences in the initial conformations. For example, in the technique of evaluating physical properties using the results of molecular simulation described above, the initial conformational dependence of the calculation results is not taken into account. Moreover, the technique for improving the accuracy of physical property calculation using the temporal fluctuation (time variation) of molecular dynamics simulation is a method subject to a single simulation, and the initial conformational dependence is also not taken into account.

Furthermore, in the technique for classifying the calculation results into favorable and unfavorable ones using the RMSD of the three-dimensional structure of the molecule as an index, it is difficult to appropriately take into account the influence of the calculation results of solubility features on the initial conformation, in the classification using the RMSD that indicates the similarity of the whole molecule. Therefore, even when this technique is used, it is difficult to appropriately evaluate the initial conformation, and suppress the variation in the calculation results due to differences in the initial conformation.

One aspect of the present invention is to appropriately evaluate the initial conformation, and suppress variation in the calculation results of solubility features due to differences in the initial conformation.

### SUMMARY

It is an object of the present invention to at least partially solve the problems in the conventional technology.

According to an aspect of an embodiment, a solubility evaluation device, including: an initial conformation setting unit that sets multiple different initial conformations with a molecule; a molecular simulation execution unit that executes a molecular simulation on each of the initial conformations set by the initial conformation setting unit; a solubility feature calculation unit that calculates a solubility feature based on an execution result of the molecular simulation performed by the molecular simulation execution unit; a partial three-dimensional structural information acquisition unit that calculates partial three-dimensional structural information indicating one or multiple three-dimensional partial structures of the molecule with respect to each of the molecular simulation; a partial structure specification unit that specifies the partial structure corresponding to variation in the solubility feature, based on the solubility feature calculated by the solubility feature calculation unit and the partial three-dimensional structural information calculated by the partial three-dimensional structural information acquisition unit;
a thermodynamic stability evaluation unit that, by creating a group by classifying the solubility feature based on the partial structure specified by the partial structure specification unit, evaluates thermodynamic stability for each of the group; a variation evaluation unit that selects one or some of the groups based on the evaluation by the thermodynamic stability evaluation unit and that calculates variation in the solubility feature in the selected group; a variation determination unit that, when the variation calculated by the variation evaluation unit is equal to or greater than a target variation value, causes the initial conformation setting unit to set multiple new initial conformations; and an output unit that, when the variation calculated by the variation evaluation unit is less than the target variation value, outputs an evaluation result of solubility based on the solubility feature used to calculate the variation in such a case.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a block diagram of a solubility evaluation device according to an embodiment;
FIG. 2 is a diagram illustrating an example of partial three-dimensional structural information;
FIG. 3 is a diagram illustrating an example of a relation between initial conformation, solubility features, and partial three-dimensional structural information;
FIG. 4 is a diagram illustrating evaluation results of variations in solubility features;
FIG. 5 is a flowchart of a troubleshooting support process performed by the solubility evaluation device according to the embodiment;
FIG. 6 is a diagram illustrating an example of evaluation results of solubility features provided by the solubility evaluation device according to the embodiment; and
FIG. 7 is a hardware configuration diagram of the solubility evaluation device.

### DESCRIPTION OF THE EMBODIMENT

Hereinafter, an embodiment of a solubility evaluation device, a solubility evaluation method, and a solubility evaluation program will be described in detail with reference to the accompanying drawings. It is to be noted that the same reference numerals denote the same components, and overlapping description will be omitted as appropriate. Moreover, the embodiments may be appropriately combined within a consistent range.

### Overall Configuration

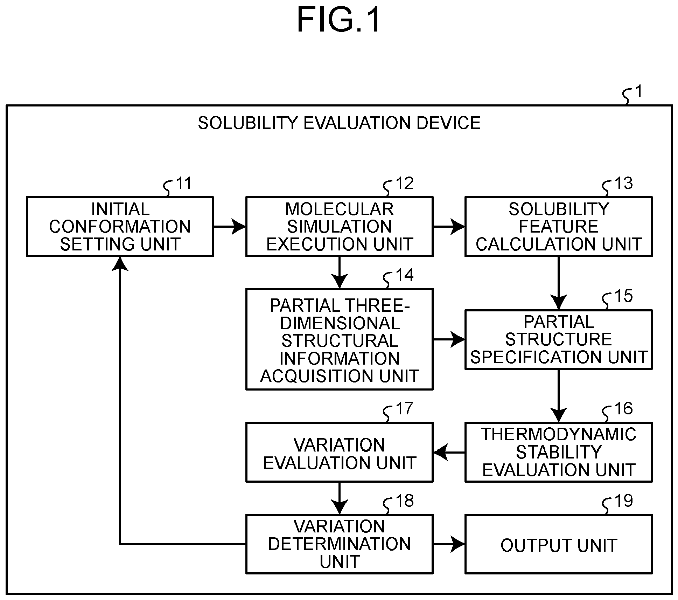

FIG. 1 is a block diagram of a solubility evaluation device according to an embodiment. For example, a solubility evaluation device 1 is a device that evaluates the solubility of a compound obtained by combining solute and solvent used in flow synthesis.

For example, in the flow synthesis, a narrow flow channel of few millimeters or less is used. Hence, the blockage of the flow channel primarily caused by low solubility of reactants and products is a major operational problem. In general, the solubility of a compound is determined by quantifying the concentration of solution at saturation, by actually conducting a solubility test.

On the other hand, if multiple reactions are required to totally synthesize the final target such as peptide synthesis, the number of combinations of solute and solvent increases with a power of the number of reaction steps. In addition, for the solubility test, a certain amount or more of solute must be prepared in advance. Hence, there is a need to separately synthesize the intermediates at each reaction step. Therefore, it is not realistic to conduct a solubility test on all the intermediates that may be produced by the flow synthesis, in terms of development man-hours and a period of time required for testing. Thus, a solubility prediction technique that does not use solubility tests has been desired.

Moreover, a solubility prediction method using computational chemistry is a method for analyzing the solvent effects by the intermolecular interaction between solute and solvent based on a statistical thermodynamic theory, and calculates the solubility features including solvation free energy. Calculation methods in solubility prediction include quantum chemical calculations such as a molecular dynamics method, a molecular orbital method, and a density functional method. To calculate the physical quantities including the solubility features without fail, it is preferable to reduce variation in the calculation results of solubility features depending on the initial conformation. In particular, the solubility features including the solvation free energy are physical quantities determined by the interaction between multiple molecular species of solute and solvent. Hence, the value of the solubility feature may change significantly by three-dimensional difference in a part of the partial structure. Therefore, the introduction of a method for specifying the partial structure corresponding to variation in the calculation results of solubility features is expected to contribute to evaluation and reduction in variation derived from the initial conformational dependence of solubility features.

Thus, the solubility evaluation device 1 according to the present embodiment implements the solubility prediction technique that does not use solubility tests with which the variation in the calculation results of solubility features derived from the initial conformational dependence of solubility features is reduced. In the following, the variation in the calculation results of solubility features is referred to as variation in the solubility features.

As illustrated in FIG. 1, the solubility evaluation device 1 includes an initial conformation setting unit 11, a molecular simulation execution unit 12, a solubility feature calculation unit 13, a partial three-dimensional structural information acquisition unit 14, a partial structure specification unit 15, a thermodynamic stability evaluation unit 16, a variation evaluation unit 17, a variation determination unit 18, and an output unit 19.

The initial conformation setting unit 11 has information on solvent and solute whose solubility is to be evaluated. For example, when compounds are synthesized through multiple reaction steps using flow synthesis, the initial conformation setting unit 11 keeps information on the combination of solvent and solute that can be used in each reaction step. In reality, the initial conformation setting unit 11 sets the following initial conformation on all the combinations of solvents and solutes. However, in the following explanation, a specific solvent and a specific solute among all the combinations will be described.

The initial conformation setting unit 11 sets a solution system containing solute and solvent molecules with the number of molecules, and sets multiple different initial conformations. The initial conformation setting unit 11 generates an initial conformation of each molecule by random numbers. For example, the initial conformation setting unit 11 generates different initial conformations by changing the structure of a functional group of the target molecule. More specifically, for example, when the molecule has a carboxyl group as a functional group, the initial conformation setting unit 11 can generate different initial conformations, by changing the relative position of a hydroxyl group relative to the double bond of carbon and oxygen. Then, the initial conformation setting unit 11 outputs the generated initial conformation group to the molecular simulation execution unit 12.

Subsequently, if it is determined by evaluation of variations in solubility features, which will be described below, that variation in solubility features is large, the initial conformation setting unit 11 receives an instruction to reset the initial conformation from the variation determination unit 18. Then, the initial conformation setting unit 11 generates multiple initial conformations other than the previously set initial conformation group at random, adds the generated initial conformations to the previously set initial conformation group, and outputs the initial conformation to the molecular simulation execution unit 12. In this manner, the initial conformation setting unit 11 increases the initial conformations and repeats the molecular simulation, until the variation in the solubility features is small.

The molecular simulation execution unit 12 receives the information on the initial conformation group, input from the initial conformation setting unit 11. Next, the molecular simulation execution unit 12 executes a molecular simulation for each initial conformation. Then, the molecular simulation execution unit 12 outputs the results of molecular simulation for each initial conformation, to the solubility feature calculation unit 13. Moreover, the molecular simulation execution unit 12 outputs the information on the initial conformation used in each molecular simulation, to the partial three-dimensional structural information acquisition unit 14.

The solubility feature calculation unit 13 receives the results of molecular simulation for each initial conformation, input from the molecular simulation execution unit 12. Then, the solubility feature calculation unit 13 calculates solubility features using the results of molecular simulation. The solubility feature is an index indicating how much solute and solvent are dissolved with each other.

For example, the solubility feature calculation unit 13 can use solvation free energy and solubility free energy as solubility features. For example, by performing molecular dynamics simulation as molecular simulation, and by using the results of molecular dynamics calculation, the solubility feature calculation unit 13 can calculate the free energy such as the solvation free energy and solubility free energy, by a free energy calculation method. For example, as the free energy calculation method, the solubility feature calculation unit 13 can use thermodynamic integration, free energy perturbation, energy display method, or the like. Then, the solubility feature calculation unit 13 outputs the solubility features that are the calculation results of each molecular simulation, to the partial structure specification unit 15.

The partial three-dimensional structural information acquisition unit 14 receives the information on the initial conformation used in simulation for each molecular simulation, input from the molecular simulation execution unit 12. Then, the partial three-dimensional structural information acquisition unit 14 calculates the partial three-dimensional structural information that represents the three-dimensional partial structure of the molecule in the results of molecular simulation for each initial conformation. The partial three-dimensional structural information may include information on a single partial structure or information on multiple partial structures. In this manner, for each initial conformation, the partial three-dimensional structural information acquisition unit 14 acquires a part but not all of the three-dimensional structure of the molecule.

For example, the partial three-dimensional structural information acquisition unit 14 can use information on the distribution of dihedral angles in the partial structure of the molecule, distribution of trihedral angles, and angle in the three-dimensional space such as bond angle, as the partial three-dimensional structural information. In addition to the above, the partial three-dimensional structural information acquisition unit 14 may also use the distance between the atoms within the partial structure of the molecule or between the partial structures, as the partial three-dimensional structural information. The partial three-dimensional structural information acquisition unit 14 can calculate multiple pieces of the partial three-dimensional structural information according to the number of molecular structures of the target molecule.

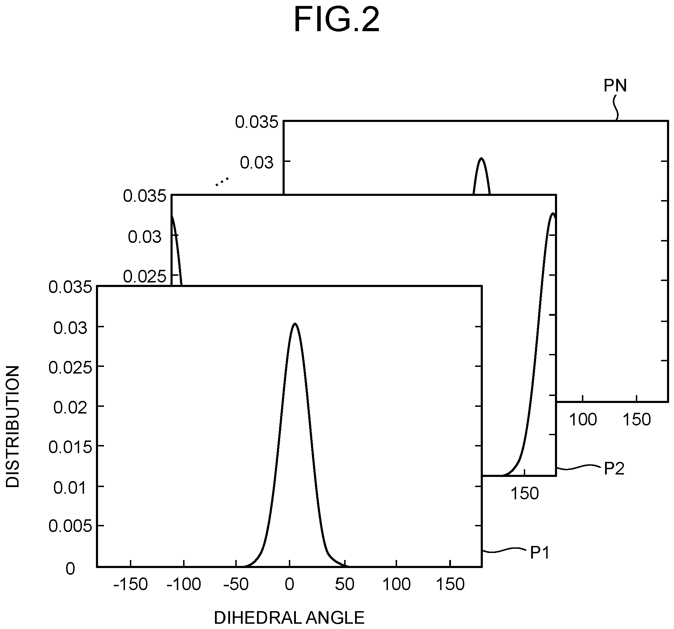

FIG. 2 is a diagram illustrating an example of partial three-dimensional structural information. For example, the partial three-dimensional structural information acquisition unit 14 acquires the distribution of dihedral angles of N pieces of partial structures from the partial structures P1 to PN illustrated in FIG. 2 with respect to the results of a single molecular simulation, as the partial three-dimensional structural information. In the graphs of partial structures P1 to PN in FIG. 2, the horizontal axis represents the dihedral angle and the vertical axis represents the probability distribution. The partial structures P1 to PN each represent a different partial structure of a single molecule. In the following, the partial three-dimensional structural information acquisition unit 14 calculates the distribution of dihedral angles as the partial three-dimensional structural information on the initial conformation of the target molecule.

In this manner, the partial three-dimensional structural information acquisition unit 14 calculates one or multiple distributions of dihedral angles corresponding to each of the results of molecular simulation. Then, the partial three-dimensional structural information acquisition unit 14 outputs the distribution of dihedral angles that is the partial three-dimensional structural information on the initial conformation for each result of molecular simulation, to the partial structure specification unit 15.

The partial structure specification unit 15 receives the solubility feature for each result of molecular simulation, input from the solubility feature calculation unit 13. Moreover, the partial structure specification unit 15 receives the distribution of dihedral angles that is the partial three-dimensional structural information on the initial conformation for each result of molecular simulation, input from the partial three-dimensional structural information acquisition unit 14. Then, the partial structure specification unit 15 specifies the partial structure corresponding to variation in the solubility features generated by depending on the initial conformation, by the multiple regression analysis in which the partial three-dimensional structural information is used as an explanatory variable and the solubility feature is used as an objective variable. For example, the partial structure specification unit 15 performs statical analysis using the average value of the distribution of the dihedral angles as the explanatory variable, or performs multiple regression analysis by machine learning, and specifies the partial structure on the basis of the distribution of the dihedral angles correlated with the solubility feature.

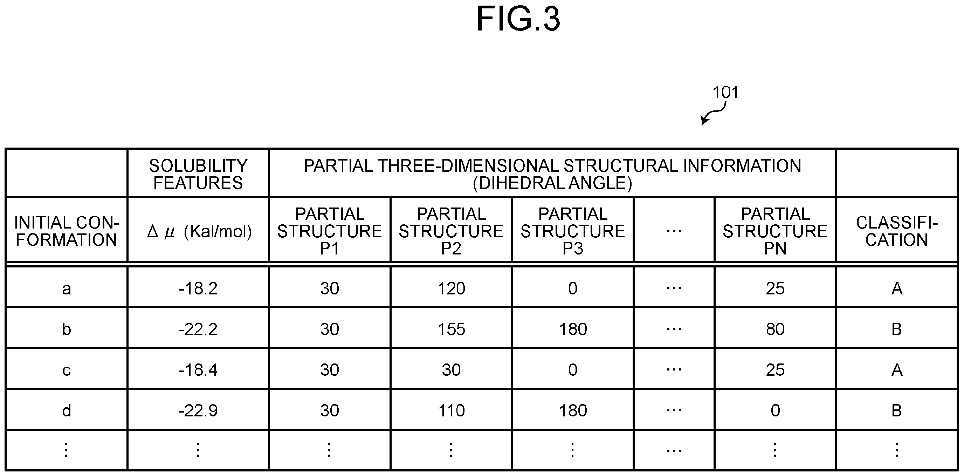

FIG. 3 is a diagram illustrating an example of a relation between initial conformation, solubility features, and partial three-dimensional structural information. In FIG. 3, the solubility feature is represented as Δµ. As illustrated in Table 101 in FIG. 3, each initial conformation has the solubility feature Δµ and partial structures P1 to PN. Hereinafter, specification of a partial structure using the distribution of dihedral angles performed by the partial structure specification unit 15 will be described in detail.

The partial structure specification unit 15 calculates the average value of the distribution of dihedral angles of each of the partial structures P1 to PN. Then, the partial structure specification unit 15 specifies the partial structure corresponding to variation in the solubility features Δµ, on the basis of the average value of the calculated distribution of dihedral angles. For example, the partial structure specification unit 15 selects the partial structure having the largest regression coefficient in the multiple regression analysis, by using the solubility feature Δµ as the objective variable, and using the average value of the distribution of dihedral angles for each partial structure as the explanatory variable. Moreover, for example, the partial structure specification unit 15 may select multiple partial structures corresponding to variation in the solubility features Δµ, and select a predetermined number of partial structures having the regression coefficient with a predetermined regression coefficient threshold or more. A large regression coefficient means a strong impact on the objective variable. Hence, by selecting the partial structure with a large regression coefficient, the partial structure specification unit 15 can select a partial structure that has a strong impact on the variation in the solubility features Δµ.

Next, as illustrated in the items classified in Table 101, the partial structure specification unit 15 classifies the results of molecular simulations for each initial conformation, on the basis of the average value of the dihedral angles of the partial structure corresponding to the value of the solubility feature Δµ. For example, in Table 101, the partial structure specification unit 15 selects the partial structure P3 as the partial structure corresponding to variation in the solubility features Δµ for each initial conformation. In this case, the partial structure specification unit 15 specifies that the value of an initial conformation a and an initial conformation c of the partial structure P3 is 0, and the value of an initial conformation b and an initial conformation d of the partial structure P3 is 180. Then, the partial structure specification unit 15 classifies the results of molecular simulation of the initial conformation a and the initial conformation c into a group A, and classifies the results of molecular simulation of the initial conformation b and the initial conformation d into a group B. Then, the partial structure specification unit 15 outputs the classification results of the results of molecular simulation of the initial conformation, to the thermodynamic stability evaluation unit 16. Moreover, the partial structure specification unit 15 outputs information on the initial conformation of the results of each molecular simulation, to the thermodynamic stability evaluation unit 16.

The thermodynamic stability evaluation unit 16 receives the classification results of the results of molecular simulation, and the information on the initial conformation of the results of each molecular simulation, input from the partial structure specification unit 15. Next, the thermodynamic stability evaluation unit 16 evaluates the thermodynamic stability for each classified group. For example, the thermodynamic stability evaluation unit 16 calculates the thermodynamic stability, using the sum of the solvation free energy and the internal free energy of the molecule.

The thermodynamic stability is a value indicating how easily the partial structure can be established for each classification. The solvation free energy is a value based on the solubility feature Δµ. The solvation free energy indicates the stability of the interaction between solute and solvent. The internal free energy of the molecule is a value obtained by performing molecular simulation on a solute molecule alone, and is a value representing the stability of the three-dimensional structure of the solute. In this case, the thermodynamic stability is increased with an increase in the negative direction. That is, the molecule in such a state can be more easily implemented. That is, by selecting the partial three-dimensional structure of the molecule determined to have a higher thermodynamic stability, the thermodynamic stability evaluation unit 16 can select the results of molecular simulation with the partial three-dimensional structure of the molecule that can be implemented more easily. The thermodynamic stability evaluation unit 16 outputs information on the thermodynamic stability for each calculated and classified group, to the variation evaluation unit 17.

The variation evaluation unit 17 receives the classification results of the results of molecular simulation, and the information on the thermodynamic stability for each group, input from the thermodynamic stability evaluation unit 16. Next, the variation evaluation unit 17 selects the group the thermodynamic stability of which evaluated by the thermodynamic stability evaluation unit 16 is the most superior, as the group with the most stable structure. By selecting the group with the most superior thermodynamic stability, the variation evaluation unit 17 can select the most feasible group, that is, the group in which the partial three-dimensional structure of the molecule is likely to exist most when solvent and solute are mixed with each other.

Then, the variation evaluation unit 17 evaluates variation σ_{Δµ} in the solubility feature Δµ of the group with the most stable structure. For example, the variation evaluation unit 17 can use a standard error derived from the difference in the initial conformation in the group with the most stable structure and the like, as σ_{Δµ}. Then, the variation evaluation unit 17 outputs the variation σ_{Δµ} in the solubility feature Δµ of the group with the most stable structure and the results of molecular simulation, to the variation determination unit 18.

In the present embodiment, the variation evaluation unit 17 evaluates the variation σ_{Δµ} in the solubility feature Δµ by selecting the group with the most stable structure. However, the variation evaluation unit 17 may also select several groups. For example, when the thermodynamic stability is superior to a predetermined stability threshold, the variation evaluation unit 17 may determine that the group is sufficiently stable thermodynamically, and select the group the thermodynamic stability of which is superior to the stability threshold. In addition to the above, the variation evaluation unit 17 may also select a predetermined number of groups such as the top two or three groups.

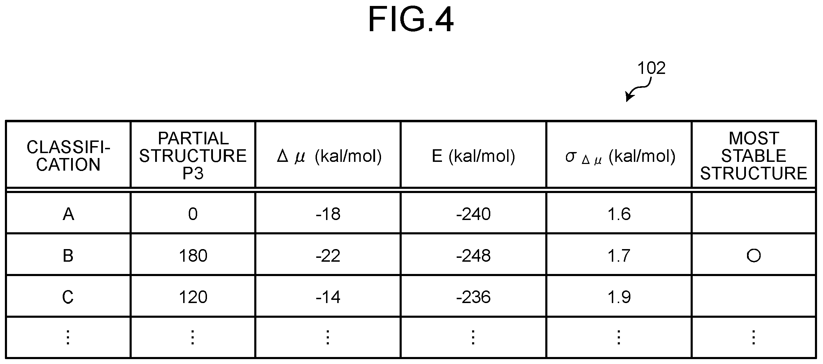

FIG. 4 is a diagram illustrating evaluation results of variations in solubility features. In Table 102 illustrated in FIG. 4, the thermodynamic stability is represented by E. As illustrated in Table 102, when the thermodynamic stability E of each group is obtained, the variation evaluation unit 17 selects a group B with the highest thermodynamic stability E in the negative direction, as a group with the most stable structure. Then, the variation evaluation unit 17 calculates the variation σ_{Δµ} in the solubility feature Δµ of the group B to be 1.7. In the example in FIG. 4, the variations σ_{Δµ} of the solubility features Δµ of the other groups are also illustrated. However, when the variation σ_{Δµ} in the solubility feature Δµ of the group with the most stable structure is calculated, there is no need to calculate the variations σ_{Δµ} of the solubility features Δµ of the other groups.

The variation determination unit 18 receives the variation σ_{Δµ} in the solubility feature Δµ of the group with the most stable structure and the results of molecular simulation, input from the variation evaluation unit 17. The variation determination unit 18 has a target variation value σ_{Δµ-set} in advance. Then, the variation determination unit 18 determines whether the variation σ_{Δµ} in the solubility feature Δµ of the group with the most stable structure obtained by the evaluation of the variation evaluation unit 17 is less than the target variation value σ_{Δµ-set}.

When the variation σ_{Δµ} in the solubility feature Δµ of the group with the most stable structure is equal to or greater than the target variation value σ_{Δµ-set}, the variation determination unit 18 determines that the variation in the solubility feature Δµ is large. Then, the variation determination unit 18 instructs the initial conformation setting unit 11 to reset the initial conformation.

In contrast, when the variation σ_{Δµ} in the solubility feature Δµ of the group with the most stable structure is less than the target variation value σ_{Δµ-set}, the variation determination unit 18 determines that the variation in the solubility feature Δµ is small. Then, the variation determination unit 18 outputs the information on the solubility feature Δµ to the output unit 19, along with the information on solvent and solute.

In the present embodiment, the initial conformation is increased at random. However, other methods may also be used to increase the initial conformation. For example, the variation determination unit 18 may also set the group with the most stable structure as a target group in which information on the initial conformation is to be increased, and instruct the initial conformation setting unit 11 to reset the initial conformation, with the information on the target group in which the information on the initial conformation is to be increased. Consequently, the initial conformation setting unit 11 can selectively increase the initial conformation having a specific structure as the structure of the group with the most stable structure.

The output unit 19 receives the solubility features of the group with the most stable structure input from the variation determination unit 18, along with the information on solvent and solute. Then, the output unit 19 provides the user with the evaluation results of solubility on the basis of the solubility features of the group with the most stable structure, by associating the evaluation results with the information on solvent and solute, by causing a display device to display the evaluation result, and the like.

The output unit 19 can provide information representing the relation between solvent and solute such as solvation free energy, as a solubility feature. For example, the output unit 19 may display the solvation free energy of each solvent with respect to a certain solute, on the display device in a comparable manner.

### Flow of Solubility Evaluation Process

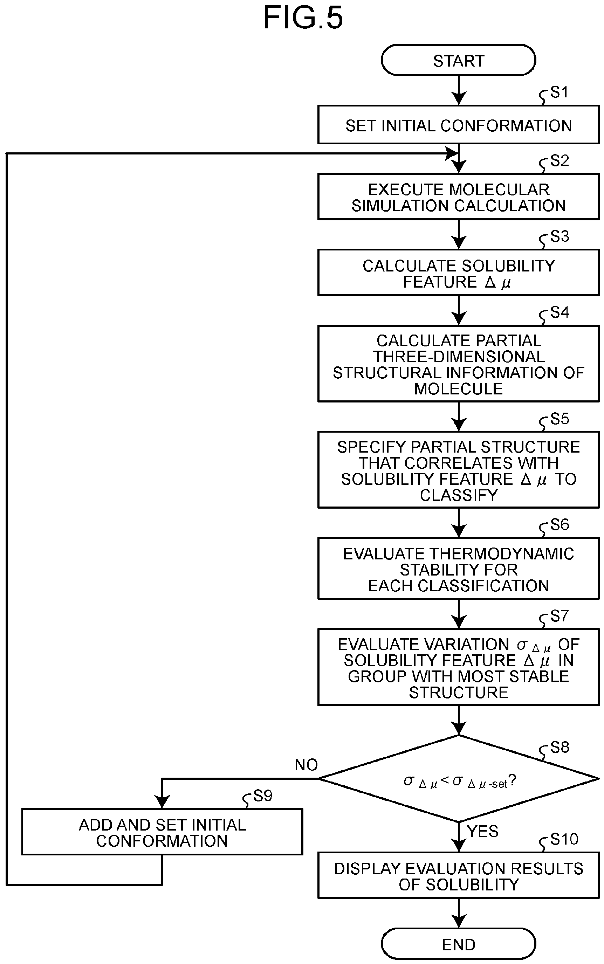

FIG. 5 is a flowchart of a troubleshooting support process performed by the solubility evaluation device according to the embodiment. Next, with reference to FIG. 5, the flow of a solubility evaluation process performed by the solubility evaluation device 1 according to the present embodiment will be described.

The initial conformation setting unit 11 sets a solution system composed of solute and solvent molecules using a random number along with the number of molecules, and sets multiple different initial conformations (step S 1).

The molecular simulation execution unit 12 obtains the initial conformation group generated by the initial conformation setting unit 11. Next, the molecular simulation execution unit 12 executes the molecular simulation calculation for each initial conformation (step S2).

The solubility feature calculation unit 13 receives the results of molecular simulation for each initial conformation, input from the molecular simulation execution unit 12. Then, the solubility feature calculation unit 13 calculates the solubility feature Δµ using the results of the molecular simulation (step S3).

The partial three-dimensional structural information acquisition unit 14 receives the information on the initial conformation used for each molecular simulation, input from the molecular simulation execution unit 12. Then, for each result of molecular simulation, the partial three-dimensional structural information acquisition unit 14 calculates the partial three-dimensional structural information that represents a partial structure of the molecule in the results of molecular simulation (step S4).

The partial structure specification unit 15 receives the solubility feature for each result of molecular simulation, input from the solubility feature calculation unit 13. Moreover, the partial structure specification unit 15 receives the partial three-dimensional structural information in the results of molecular simulation for each initial conformation, input from the partial three-dimensional structural information acquisition unit 14. Then, the partial structure specification unit 15 specifies the partial structure that correlates with the variation in the solubility features Δµ from the partial three-dimensional structural information. Next, on the basis of the partial structure corresponding to the value of the solubility feature Δµ, the partial structure specification unit 15 classifies the results of molecular simulations for each initial conformation (step S5).

The thermodynamic stability evaluation unit 16 receives the classification results of the results of molecular simulation, and the information on the initial conformation of the results of each molecular simulation, input from the partial structure specification unit 15. Next, the thermodynamic stability evaluation unit 16 evaluates the thermodynamic stability for each classified group (step S6).

The variation evaluation unit 17 receives the classification results of the results of molecular simulation, and the information on the thermodynamic stability for each group, input from the thermodynamic stability evaluation unit 16. Next, the variation evaluation unit 17 selects the group the thermodynamic stability of which evaluated by the thermodynamic stability evaluation unit 16 is the most superior, as the group with the most stable structure. Then, the variation evaluation unit 17 evaluates the variation σ_{Δµ} in the solubility feature Δµ of the group with the most stable structure (step S7).

The variation determination unit 18 receives the variability σ_{Δµ} of the group with the most stable structure and the results of molecular simulation, input from the variation evaluation unit 17. Next, the variation determination unit 18 determines whether the variation σ_{Δµ} of the group with the most stable structure is less than the target variation value σ_{Δµ-set} (step S8).

When the variation σ_{Δµ} in the solubility feature Δµ of the thermodynamic stability of the group with the most stable structure is equal to or greater than the target variation value σ_{Δµ-set} (No at step S8), the variation determination unit 18 instructs the initial conformation setting unit 11 to reset the initial conformation. The initial conformation setting unit 11 receives the instruction to reset the initial conformation from the variation determination unit 18, and sets a new initial conformation, by generating multiple new initial conformations at random, and adding the new initial conformations to the previously generated initial conformation group (step S9). Then, the solubility evaluation process returns to step S2.

In contrast, when the variation σ_{Δµ} in the solubility feature Δµ of the group with the most stable structure is less than the target variation value σ_{Δµ-set} (Yes at step S8), the variation determination unit 18 determines that the variation in the solubility feature Δµ is small. Then, the variation determination unit 18 outputs the results of molecular simulation to the output unit 19, along with the information on solvent and solute. The output unit 19 receives the solubility feature Δµ of the group with the most stable structure input from the variation determination unit 18, along with the information on solvent and solute. Then, the output unit 19 provides the user with the evaluation results of solubility on the basis of the solubility feature Δµ of the group with the most stable structure corresponding to the information on solvent and solute, by displaying the evaluation results on a display device or the like (step S10).

### Effects

As described above, the solubility evaluation device according to the embodiment executes a molecular simulation to evaluate solubility, by setting multiple initial conformations for the combination of solvent and solute, and classifies the results of molecular simulations by a part of the partial structure of the molecule. Next, the solubility evaluation device according to the embodiment specifies the group with the highest thermodynamic stability in the classified group, and evaluates the variation in the results of molecular simulation in the group. Then, when the variation is equal to or greater than the threshold, the solubility evaluation device resets the initial conformation by adding the initial conformation and the like, and repeats the molecular simulation and the evaluation on the variation in the results of molecular simulation, until the variation falls under the threshold.

Consequently, it is possible to obtain the results of molecular simulation, by performing molecular simulation using the initial conformation that is most likely present and that can suppress the variation in the results of molecular simulation. Consequently, it is possible to perform an appropriate solubility evaluation, by appropriately evaluating the results of molecular simulation with respect to the initial conformation, and suppressing the variation in the calculation results due to differences in the initial conformation. Therefore, it is possible to improve the accuracy of solubility prediction using computational chemistry.

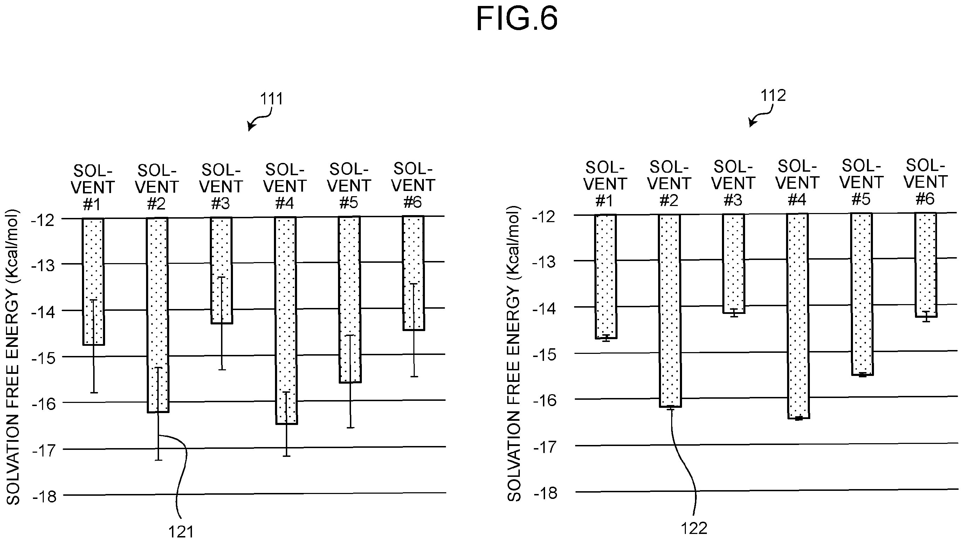

FIG. 6 is a diagram illustrating an example of evaluation results of solubility features provided by the solubility evaluation device according to the embodiment. A graph 111 in FIG. 6 is a diagram illustrating the evaluation results of solubility features when the initial conformation is not selected. Moreover, a graph 112 is a diagram illustrating the evaluation results of solubility features when the solubility evaluation device 1 according to the present embodiment is used.

The graphs 111 and 112 are graphs representing the solvation free energy of solution containing a specific solute and solvents #1 to #6 calculated by computational chemistry. In the graphs 111 and 112, the horizontal axis represents the type of solvent, and the vertical axis represents the solvation free energy. Moreover, in the graphs 111 and 112, stability is improved in the downward direction. For example, the output unit 19 of the solubility evaluation device 1 according to the present embodiment causes the display device to display the graph 112.

For example, a line segment attached to the tip of the band representing the solvation free energy of each solvent #1 to #6 such as a line segment 121 in the graph 111, represents the range of variation in the solvation free energy calculated for each solvent #1 to #6. Similarly, each line segment such as a line segment 122 in the graph 112 also represents the range of variation in the solvation free energy calculated for each solvent #1 to #6. The variations in the graph 112 are smaller than the variations in the graph 111. For example, in the graph 111, the solvation free energy of the solvent #2 is possibly larger than that of the solvent #4 in the negative direction. In contrast, in the graph 112, it is possible to confirm that the solvation free energy of the solvent #2 is smaller than that of the solvent #4 in the negative direction. Therefore, for example, when selecting between the solvent #2 and the solvent #4, it is possible to select the solvent with high stability without fail, by using the graph 112 provided by the solubility evaluation device 1 according to the present embodiment. For example, if there are ten reaction steps in the synthesis, it is possible to appropriately evaluate the stability at each reaction step, and select the optimal route with high solubility.

In the example in FIG. 6, dependency on the soluble solvent is indicated by fixing the solute. However, the solubility evaluation device 1 according to the present embodiment can also provide information indicating the dependency on the soluble solute by fixing the solvent. However, when the solvent is fixed, the meltability may change according to the solid state of the solute. Therefore, to provide information indicating the dependency on the soluble solute by fixing the solvent, it is preferable that the solubility evaluation device 1 evaluate the solubility feature while taking into account the solid state of the solute, and provide evaluation on the evaluation results.

As a method for analyzing the calculation results of molecular dynamics, there is a technique for classifying the calculation results into favorable and unfavorable ones, using the RMSD of the three-dimensional structure of the molecule as an index. However, in this technology, as the information on the three-dimensional structure of the molecule, the RMSD indicating the similarity of the three-dimensional structure of the whole molecule is used. In two three-dimensional structures, the distance between the corresponding two points is defined as a square root of the arithmetic means. Hence, although the RMSD is often used as an index of similarity in the entire three-dimensional structure, the RMSD is not suitable for extracting differences in the specific partial structure. The partial structure of the molecule affects largely on the variation in the solubility features. Hence, to reduce the variation in the calculation results due to the difference in the initial conformation, it is effective to specify the partial structure corresponding to variation in the calculation results, and classify the calculation results for each partial structure. When classification is performed only by using the RMSD indicating the similarity of the whole molecule as the three-dimensional structural information, and when the partial structure is not taken into account, it is difficult to perform classification based on the molecular structure according to the variation in the solubility features.

In contrast, the solubility evaluation device according to the present embodiment specifies the three-dimensional partial structure of the molecule corresponding to variation in the calculation results, and classifies each partial structure. Hence, the solubility evaluation device can easily specify the structure that suppresses the variation in the solubility features. Therefore, unlike the techniques that classify molecules using the RMSD as an index, the solubility evaluation device according to the present embodiment can perform an appropriate solubility evaluation, by appropriately evaluating the initial conformation, and suppressing the variation in the calculation results due to differences in the initial conformation.

### System

The processing procedures, control procedures, specific names, and information including various types of data and parameters illustrated in the above documents and drawings may be optionally changed unless otherwise specified.

Moreover, each component of each device illustrated in the drawings is functionally conceptual and need not necessarily be physically configured as illustrated in the drawings. That is, the specific modes of distribution and integration of the devices are not limited to those illustrated in the drawings. In other words, all or part of the devices can be functionally or physically distributed or integrated in an optional unit according to various loads and the state of use.

Moreover, all or an optional part of various processing functions performed by each device can be implemented by a Central Processing Unit (CPU) and a computer program that is analyzed and executed by the CPU, or by hardware using wired logic.

### Hardware

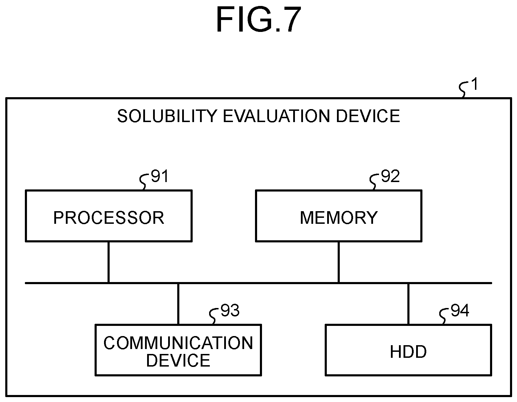

Next, a hardware configuration example of the solubility evaluation device 1 will be described. FIG. 7 is a hardware configuration diagram of the solubility evaluation device. As illustrated in FIG. 7, the solubility evaluation device 1 includes a processor 91, a memory 92, a communication device 93, and a Hard Disk Drive (HDD) 94. Moreover, the processor 91 is connected to the memory 92, the communication device 93, and the HDD 94 via a bus.

The communication device 93 is a network interface card or the like, and communicates with other information processing devices.

The HDD 94 is an auxiliary storage device. The HDD 94 stores various computer programs including computer programs for implementing the functions of the initial conformation setting unit 11, the molecular simulation execution unit 12, the solubility feature calculation unit 13, the partial three-dimensional structural information acquisition unit 14, the partial structure specification unit 15, the thermodynamic stability evaluation unit 16, the variation evaluation unit 17, the variation determination unit 18, and the output unit 19.

The processor 91 reads various computer programs stored in the HDD 94, expands the read computer programs in the memory 92, and executes the computer programs. Consequently, the processor 91 implements the functions of the initial conformation setting unit 11, the molecular simulation execution unit 12, the solubility feature calculation unit 13, the partial three-dimensional structural information acquisition unit 14, the partial structure specification unit 15, the thermodynamic stability evaluation unit 16, the variation evaluation unit 17, the variation determination unit 18, and the output unit 19.

In this manner, the solubility evaluation device 1 operates as an information processing device that performs various processing methods by reading and executing a computer program. Moreover, the solubility evaluation device 1 can implement the same function as the embodiment described above, by reading the computer program described above from the recording medium by a medium reading device, and executing the read computer program. The computer program in this example is not limited to being executed by the solubility evaluation device 1. For example, the present invention can be similarly applied to a case where another computer or server executes the computer program or when the other computer and server cooperate with each other to execute the computer program.

The computer program can be distributed via a network such as the Internet. Moreover, the computer program can be recorded on a computer-readable recording medium such as a hard disk, a flexible disk (FD), a CD-ROM, a Magneto-Optical disk (MO), and a Digital Versatile Disc (DVD), and executed by being read out from the recording medium by a computer.

Some examples of combinations of the disclosed technical features will be described below.
(1) A solubility evaluation device, includes:
   an initial conformation setting unit that sets multiple different initial conformations with a molecule;
   a molecular simulation execution unit that executes a molecular simulation on each of the initial conformations set by the initial conformation setting unit;
   a solubility feature calculation unit that calculates a solubility feature based on an execution result of the molecular simulation performed by the molecular simulation execution unit;
   a partial three-dimensional structural information acquisition unit that calculates partial three-dimensional structural information indicating one or multiple three-dimensional partial structures of the molecule with respect to each of the molecular simulation;
   a partial structure specification unit that specifies the partial structure corresponding to variation in the solubility feature, based on the solubility feature calculated by the solubility feature calculation unit and the partial three-dimensional structural information calculated by the partial three-dimensional structural information acquisition unit;
   a thermodynamic stability evaluation unit that, by creating a group by classifying the solubility feature based on the partial structure specified by the partial structure specification unit, evaluates thermodynamic stability for each of the group;
   a variation evaluation unit that selects one or some of the groups based on the evaluation by the thermodynamic stability evaluation unit and that calculates variation in the solubility feature in the selected group;
   a variation determination unit that, when the variation calculated by the variation evaluation unit is equal to or greater than a target variation value, causes the initial conformation setting unit to set multiple new initial conformations; and
   an output unit that, when the variation calculated by the variation evaluation unit is less than the target variation value, outputs an evaluation result of solubility based on the solubility feature used to calculate the variation in such a case.
(2) The solubility evaluation device according to (1), wherein the molecular simulation execution unit uses molecular dynamics simulation.
(3) The solubility evaluation device according to (1) or (2), wherein the solubility feature calculation unit uses solvation free energy as a solubility feature.
(4) The solubility evaluation device according to any one of (1) to (3), wherein, as partial three-dimensional structural information, the partial three-dimensional structural information acquisition unit uses a three-dimensional structure relating to an angle.
(5) The solubility evaluation device according to (4), wherein, as the partial three-dimensional structural information, the partial three-dimensional structural information acquisition unit uses distribution of dihedral angles.
(6) The solubility evaluation device according to any one of (1) to (5), wherein the partial structure specification unit specifies the partial structure by a regression analysis in which the partial three-dimensional structural information is used as an explanatory variable and the solubility feature is used as an objective variable.
(7) The solubility evaluation device according to (6), wherein, as the partial structural information, the partial structure specification unit performs statical analysis using an average value of distribution of dihedral angles as an explanatory variable, or performs the regression analysis by machine learning, and specifies the partial structure based on the distribution of the dihedral angles correlated with the solubility feature.
(8) The solubility evaluation device according to any one of (1) to (7), wherein the variation evaluation unit selects the group that is evaluated by the thermodynamic stability evaluation unit to be most thermodynamically stable.
(9) The solubility evaluation device according to any one of (1) to (8), wherein the initial conformation setting unit sets multiple new initial conformations by adding an additional initial conformation belonging to the group selected by the variability evaluation unit, to the initial conformations.
(10) A solubility evaluation method that causes a solubility evaluation device to execute a process, including:
   setting multiple different initial conformations with a molecule;
   executing a molecular simulation on each of the set initial conformations;
   calculating a solubility feature based on an execution result of the molecular simulation;
   calculating partial three-dimensional structural information indicating one or multiple three-dimensional partial structures of the molecule with respect to each of the molecular simulation;
   specifying the partial structure corresponding to variation in the solubility feature, based on the solubility feature and the partial three-dimensional structural information;
   by creating a group by classifying the solubility feature based on the specified partial structure, evaluating thermodynamic stability for each of the group;
   by selecting one or some of the groups based on the evaluation, calculating variation in the solubility feature in the selected group;
   when the calculated variation is equal to or greater than a target variation value, by setting multiple new initial conformations, executing a process of executing the molecular simulation, calculating the solubility feature, calculating the partial three-dimensional structural information, specifying the partial structure, evaluating the thermodynamic stability, and calculating the variation, based on the new initial conformations; and
   when the calculated variation is less than the target variation value, outputting an evaluation result of solubility based on the solubility feature used to calculate the variation in such a case.
(11) A solubility evaluation program that causes a computer to execute a process, including:
   setting multiple different initial conformations with a molecule;
   executing a molecular simulation on each of the set initial conformations;
   calculating a solubility feature based on an execution result of the molecular simulation;
   calculating partial three-dimensional structural information indicating one or multiple three-dimensional partial structures of the molecule with respect to each of the molecular simulation;
   specifying the partial structure corresponding to variation in the solubility feature, based on the solubility feature and the partial three-dimensional structural information;
   by creating a group by classifying the solubility feature based on the specified partial structure, evaluating thermodynamic stability for each of the group;
   by selecting one or some of the groups based on the evaluation, calculating variation in the solubility feature in the selected group;
   when the calculated variation is equal to or greater than a target variation value, by setting multiple new initial conformations, executing a process of executing the molecular simulation, calculating the solubility feature, calculating the partial three-dimensional structural information, specifying the partial structure, evaluating the thermodynamic stability, and calculating the variation, based on the new initial conformations; and
   when the calculated variation is less than the target variation value, outputting an evaluation result of solubility based on the solubility feature used to calculate the variation in such a case.

According to the present invention, it is possible to appropriately evaluate the results of molecular simulation with respect to the initial conformation, and suppress the variation in the calculation results of solubility features due to differences in the initial conformation.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. A solubility evaluation device, comprising:
an initial conformation setting unit that sets multiple different initial conformations with a molecule;
a molecular simulation execution unit that executes a molecular simulation on each of the initial conformations set by the initial conformation setting unit;
a solubility feature calculation unit that calculates a solubility feature based on an execution result of the molecular simulation performed by the molecular simulation execution unit;
a partial three-dimensional structural information acquisition unit that calculates partial three-dimensional structural information indicating one or multiple three-dimensional partial structures of the molecule with respect to each of the molecular simulation;
a partial structure specification unit that specifies the partial structure corresponding to variation in the solubility feature, based on the solubility feature calculated by the solubility feature calculation unit and the partial three-dimensional structural information calculated by the partial three-dimensional structural information acquisition unit;
a thermodynamic stability evaluation unit that, by creating a group by classifying the solubility feature based on the partial structure specified by the partial structure specification unit, evaluates thermodynamic stability for each of the group;
a variation evaluation unit that selects one or some of the groups based on the evaluation by the thermodynamic stability evaluation unit and that calculates variation in the solubility feature in the selected group;
a variation determination unit that, when the variation calculated by the variation evaluation unit is equal to or greater than a target variation value, causes the initial conformation setting unit to set multiple new initial conformations; and
an output unit that, when the variation calculated by the variation evaluation unit is less than the target variation value, outputs an evaluation result of solubility based on the solubility feature used to calculate the variation in such a case.

2. The solubility evaluation device according to claim 1, wherein the molecular simulation execution unit uses molecular dynamics simulation.

3. The solubility evaluation device according to claim 2 or 3, wherein the solubility feature calculation unit uses solvation free energy as a solubility feature.

4. The solubility evaluation device according to any one of claims 1 to 3, wherein, as partial three-dimensional structural information, the partial three-dimensional structural information acquisition unit uses a three-dimensional structure relating to an angle.

5. The solubility evaluation device according to claim 4, wherein, as the partial three-dimensional structural information, the partial three-dimensional structural information acquisition unit uses distribution of dihedral angles.

6. The solubility evaluation device according to any one of claims 1 to 5, wherein the partial structure specification unit specifies the partial structure by a regression analysis in which the partial three-dimensional structural information is used as an explanatory variable and the solubility feature is used as an objective variable.

7. The solubility evaluation device according to claim 6, wherein, as the partial structural information, the partial structure specification unit performs statical analysis using an average value of distribution of dihedral angles as an explanatory variable, or performs the regression analysis by machine learning, and specifies the partial structure based on the distribution of the dihedral angles correlated with the solubility feature.

8. The solubility evaluation device according to any one of claims 1 to 7, wherein the variation evaluation unit selects the group that is evaluated by the thermodynamic stability evaluation unit to be most thermodynamically stable.

9. The solubility evaluation device according to any one of claims 1 to 8, wherein the initial conformation setting unit sets multiple new initial conformations by adding an additional initial conformation belonging to the group selected by the variability evaluation unit, to the initial conformations.

10. A solubility evaluation method that causes a solubility evaluation device to execute a process, comprising:
setting multiple different initial conformations with a molecule;
executing a molecular simulation on each of the set initial conformations;
calculating a solubility feature based on an execution result of the molecular simulation;
calculating partial three-dimensional structural information indicating one or multiple three-dimensional partial structures of the molecule with respect to each of the molecular simulation;
specifying the partial structure corresponding to variation in the solubility feature, based on the solubility feature and the partial three-dimensional structural information;
by creating a group by classifying the solubility feature based on the specified partial structure, evaluating thermodynamic stability for each of the group;
by selecting one or some of the groups based on the evaluation, calculating variation in the solubility feature in the selected group;
when the calculated variation is equal to or greater than a target variation value, by setting multiple new initial conformations, executing a process of executing the molecular simulation, calculating the solubility feature, calculating the partial three-dimensional structural information, specifying the partial structure, evaluating the thermodynamic stability, and calculating the variation, based on the new initial conformations; and
when the calculated variation is less than the target variation value, outputting an evaluation result of solubility based on the solubility feature used to calculate the variation in such a case.

11. A solubility evaluation program that causes a computer to execute a process, comprising:
setting multiple different initial conformations with a molecule;
executing a molecular simulation on each of the set initial conformations;
calculating a solubility feature based on an execution result of the molecular simulation;
calculating partial three-dimensional structural information indicating one or multiple three-dimensional partial structures of the molecule with respect to each of the molecular simulation;
specifying the partial structure corresponding to variation in the solubility feature, based on the solubility feature and the partial three-dimensional structural information;
by creating a group by classifying the solubility feature based on the specified partial structure, evaluating thermodynamic stability for each of the group;
by selecting one or some of the groups based on the evaluation, calculating variation in the solubility feature in the selected group;
when the calculated variation is equal to or greater than a target variation value, by setting multiple new initial conformations, executing a process of executing the molecular simulation, calculating the solubility feature, calculating the partial three-dimensional structural information, specifying the partial structure, evaluating the thermodynamic stability, and calculating the variation, based on the new initial conformations; and
when the calculated variation is less than the target variation value, outputting an evaluation result of solubility based on the solubility feature used to calculate the variation in such a case.
